# EUROPEAN PATENT SPECIFICATION

(11) **EP 3 324 188 B1**
(45) Date of publication and mention of the grant of the patent: **09.01.2019**
(21) Application number: 17200206.5
(22) Date of filing: 06.11.2017
(51) Int. Cl.: G01N 33/68

(54) **IN VITRO DIAGNOSTIC METHOD FOR ALZHEIMER'S DISEASE BASED ON THE ALBUMIN REDOX LEVEL IN THE CEREBROSPINAL FLUID**
IN VITRO DIAGNOSTISCHES VERFAHREN FÜR ALZHEIMER-KRANKHEIT BASIEREND AUF DEN ALBUMIN REDOX SPIEGEL IN DER ZEREBROSPINALFLÜSSIGKEIT
MÉTHODE DE DIAGNOSTIC IN VITRO POUR LA MALADIE D'ALZHEIMER BASÉE SUR LE NIVEAU RÉDOX D'ALBUMINE DANS LE LIQUIDE CÉRÉBRO-SPINAL

(30) Priority: 16.11.2016 ES 201631468
(43) Date of publication of application: 23.05.2018
(73) Proprietor: Grifols Worldwide Operations Limited, Dublin 22 (IE)
(72) Inventor: GRANCHA GAMON, SALVADOR, 08401 GRANOLLERS (BARCELONA) (ES); COSTA RIEROLA, MONTSERRAT, 08302 MATARO (BARCELONA) (ES); ORTIZ FERNANDEZ, ANA MARIA, 08150 PARETS DEL VALLES (ES)
(74) Representative: Durán-Corretjer, S.L.P.

(56) References cited:
- WO-A2-2006/110621
- WO-A2-2013/024100
- ES-A1- 2 600 386
- HEAFIELD M ET AL: "Plasma cysteine and sulphate levels in patients with motor neurone, Parkinson's and Alzheimer's disease", NEUROSCIENCE LETTERS, ELSEVIER, AMSTERDAM, NL, vol. 110, no. 1-2, 2 March 1990 (1990-03-02), pages 216-220, XP025445917, ISSN: 0304-3940, DOI: 10.1016/0304-3940(90)90814-P [retrieved on 1990-03-02]
- KELD POULSEN ET AL: "Characterization and stability of transthyretin isoforms in cerebrospinal fluid examined by immunoprecipitation and high-resolution mass spectrometry of intact protein", METHODS, vol. 56, no. 2, 21 January 2012 (2012-01-21), pages 284-292, XP028472194, ISSN: 1046-2023, DOI: 10.1016/J.YMETH.2011.12.009 [retrieved on 2012-01-21]
- NAKAMURA TOMOHIRO ET AL: "Aberrant Protein S-Nitrosylation in Neurodegenerative Diseases", NEURON, CELL PRESS, US, vol. 78, no. 4, 22 May 2013 (2013-05-22), pages 596-614, XP028551433, ISSN: 0896-6273, DOI: 10.1016/J.NEURON.2013.05.005

## Description

The present invention relates to the sector of clinical diagnosis, and relates in particular to an *in vitro* method for diagnosing Alzheimer's disease (AD) based on determining the redox state of albumin in cerebrospinal fluid (CSF), in particular the content of mercaptoalbumin (HMA), which is the reduced form of albumin containing cysteine Cys-34 in the form of a free thiol group.

AD is a progressive neurodegenerative disease, typically characterised by loss of short-term memory and other mental abilities (such as higher cognitive abilities) as the neurons die and various areas of the brain become atrophied. The disease tends to have an average duration of approximately 10 years from diagnosis, although said duration can vary in direct proportion to the severity of the disease at the time of diagnosis.

AD is the most common form of dementia; it is incurable and terminal, and occurs more frequently in persons aged over 65 years although it can also, in rare cases, develop as early as the 40s.

In general, the initial symptom is the inability to acquire new memories, but it tends to be confused with aging- or stress-related behaviour. When AD is suspected, the clinical diagnosis procedures available while the patient is living have up to now been based on guidelines and criteria established by the National Institute of Neurological Disorders and Stroke-Alzheimer's Disease and Related Disorders Association (NINCDS-ADRDA) (McKhann et al., 1984), mostly based on clinical opinion according to the results of neuropsychological tests, reports from the patient's family and friends and a general neurological assessment. The neurological examination can also include a study of brain images (neuroimaging).

There is currently no definitive diagnostic method for AD, apart from post-mortem histological analysis of the patient's brain.

It is, however, known that human albumin is a non-glycosylated 66-kDa protein. Quantitatively, it is the most important protein in the blood plasma and its concentration in normal plasma is between 35 and 50 g/L, constituting up to 60% of total plasma proteins (Peters T. J.: All About Albumin; Biochemistry, Genetics, and Medical Applications. Academic Press, San Diego, 1996). Similarly, albumin is also the most abundant protein in the CSF, constituting around 67% of total proteins, with a concentration of around 200 mg/L (Edward J. Thompson, 2005, The roster of CSF proteins, PROTEINS OF THE CEREBROSPINAL FLUID: Analysis and Interpretation in the Diagnosis and Treatment of Neurological Disease, 2nd Ed. London, UK: Elsevier Academic Press.: 13-31).

The structure of human albumin consists of a polypeptide of 585 amino acids and approximately 67% alpha helices having no beta sheets (Otagiri M., Chuang V.T. Pharmaceutically important pre- and posttranslational modifications on human serum albumin. Biol Pharm Bull 2009; 32:527-534). Human albumin contains 6 methionine residues and 35 cysteine residues, the latter being involved in the formation of 17 disulfide bonds. Cys-34 is the only free cysteine in the whole molecule. Human albumin has specific antioxidant functions owing to its ability to bind to multiple ligands and its radical capture properties, both being closely related to its structure.

Although there are many possibilities for oxidising albumin, Cys-34 is a site that is particularly sensitive to oxidation/reduction. Therefore, it is generally legitimate to speak of the redox state of albumin in terms of Cys-34. Using chromatographic separation of albumin, three fractions are obtained depending on the redox state of the Cys-34 (Peters, 1996 cited above):
(i) the reduced form with cysteine in the form of a free thiol group, called human mercaptoalbumin (HMA);
(ii) the oxidised form with cysteine, forming a disulfide bond with a small compound containing a thiol group, mainly cysteine or cysteinyl-glycine, although it can also be with homocysteine and glutathione, called human non-mercaptoalbumin-1 (HNA1); and
(iii) the most oxidised form with cysteine as sulfinic or sulfonic acid, called human non-mercaptoalbumin-2 (HNA2).
In a healthy, sound person, approximately 61-69% of total albumin in plasma takes the form of HMA, 27-35% the form of HNA1 and 3-5% the form of HNA2 (Oettl K., et al. Oxidative damage of albumin in advanced liver disease. Biochim Biophys. Acta 2008; 1782: 469-473; Oettl K. and Marsche G. Redox State of Human Serum Albumin in Terms of Cysteine-34 in Health and Disease. Methods Enzymol. 2010; 474:181-95; and Oettl K. et al. Oxidative albumin damage in chronic liver failure: relation to albumin binding capacity; liver dysfunction and survival. J Hepatol, 2013, 59:978-983). It is generally believed that oxidisation of HMA and HNA1 is reversible, whereas oxidisation of HNA2 is an irreversible process. Albumin can be subjected to various structural modifications, which means that its shape is modified and, therefore, its binding properties, as well as its redox state (Oettl, K. et al., 2010 cited above)

WO 2013/024100 discloses the diagnosis, prevention or treatment of medical conditions associated with oxidative stress, in particular liver diseases, by quantifying the level of non-mercaptalbumin-2 (the fraction of albumin that is irreversibly oxidized at cysteine 34).

WO 2006/110621 discloses a method for diagnosing a subject's AD state. The method involves providing a database containing information relating to protein expression levels associated and not associated with AD. Albumin is listed, amongst other markers.

The present invention is based on the surprising discovery that, in patients diagnosed with AD while they are living, the content of the reduced form of albumin (HMA) in the cerebrospinal fluid (CSF) is much lower than in healthy control subjects of an equivalent age range. This phenomenon has not been observed for the content of HMA in blood plasma, also when compared with healthy control subjects of an equivalent age range. Hence, it can also be said that it has been discovered that in patients diagnosed with AD while they are living, the difference or ratio between the HMA level in the CSF and in the plasma is increased compared with the same difference in healthy control subjects of an equivalent age range. Therefore, the two markers mentioned above, i.e. the content of HMA in CSF and the difference or ratio between the level of HMA in CSF and plasma, are useful for diagnosing AD.
As mentioned above, the definitive diagnostic test for AD has to be carried out post-mortem, and therefore diagnosis in a living patient is based on clinical opinion according to the results of neurological tests, reports from the patient's family and friends and a general neurological assessment. The test of the present invention therefore represents a diagnostic tool that is objective, quantifiable, easy to interpret, reliable, reproducible in different diagnostic centres, relatively inexpensive and not influenced by cultural aspects such as the patient's educational level, as is the case with some neuropsychological tests.

As used in the present document, "healthy subject" and its plural refer to subjects not suffering from AD.

Therefore, in a first aspect, the present invention relates to an *in vitro* method for diagnosing Alzheimer's disease (AD), comprising the following steps:
a) determining the content of mercaptoalbumin (HMA) in a sample of cerebrospinal fluid (CSF) from a patient; and
b) comparing the content determined in a) with the content of HMA in CSF in healthy subjects.

In a preferred embodiment, in step b) of the method of the present invention, if the HMA content determined in step a) is less than that of healthy subjects, it is indicative of AD; preferably the patient is diagnosed with AD.

In a preferred embodiment, both the patient and the healthy subjects mentioned above are human beings, preferably adult human beings.

In another preferred embodiment, the content of HMA in CSF is measured by high-performance liquid chromatography (HPLC) and fluorescence detection (FLD) using excitation and emission wavelengths of 280 and 340 nm respectively, based on the methodology described by Oettl K., 2010 (see above). HMA is quantified by taking account of the height of the relevant peak obtained in the corresponding chromatogram.

The cut-off value, determined by calculating the ROC curve after adjusting the sensitivity and specificity percentages to maximum values (91% and 100% respectively), below which it is considered that the HMA content measured in step a) is indicative of AD and/or leads to a diagnosis of AD in the patient, is preferably 37% (w/v).

In a second embodiment, the present invention relates to an *in vitro* method for diagnosing Alzheimer's disease (AD), comprising the following steps:
a) determining the content of mercaptoalbumin (HMA) in a sample of cerebrospinal fluid (CSF) and a sample of blood or plasma from a patient;
b) determining a difference or ratio between the content of HMA in the sample of CSF and the content of HMA in the sample of blood or plasma determined in step a); and
c) comparing the difference or ratio determined in step b) with the corresponding difference or ratio in healthy subjects.

In step b) of the method according to this second embodiment, it is envisaged that the difference determined is: the difference between the content of HMA in the sample of CSF and the content of HMA in the sample of blood or plasma determined in step a); or the difference between the content of HMA in the sample of blood or plasma and the content of HMA in the sample of CSF determined in step a). In a preferred embodiment, in step b) the difference between the content of HMA in the sample of CSF and the content of HMA in the sample of blood or plasma determined in step a) is determined in the form of a ratio (value of HMA in plasma/value of HMA in CSF).

When in step b) the difference is determined between the content of HMA in the sample of CSF and the content of HMA in the sample of blood or plasma determined in step a), in a preferred embodiment, in step c) of the method of the present invention, if the difference determined in step b), for example the ratio of the value of HMA in plasma / value of HMA in CSF (HMA plasma/HMA CSF), is greater than that of healthy subjects, it is indicative of AD; more preferably, the patient is diagnosed with AD. If the difference determined is something else, a person skilled in the art will make the necessary adaptations to the method of the present invention (for example, in relation to the comparison with the corresponding value of the difference in healthy subjects).

Additionally, and preferably, the sample of blood or plasma is a sample of plasma.

In a preferred embodiment, both the patient and the healthy subjects mentioned above are human beings, preferably adult human beings.

In another preferred embodiment, the content of HMA in CSF is measured by high-performance liquid chromatography (HPLC) and fluorescence detection (FLD) using excitation and emission wavelengths of 280 and 340 nm respectively, based on the methodology described by Oettl K., 2010 (see above). HMA is quantified by taking account of the height of the relevant peak obtained in the corresponding chromatogram.

The cut-off value, determined by calculating the ROC curve after adjusting the sensitivity and specificity percentages to maximum values (100%), from which it is considered that the difference determined in step b), for example the ratio of the value of HMA in plasma / value of HMA in CSF (HMA plasma/HMA CSF), is indicative of AD and/or that it leads to the diagnosis of AD in the patient, is preferably 1.1.

To aid understanding, the present invention is described in greater detail below, with reference to the attached figures, which are presented by way of example, and with reference to illustrative but nonlimiting examples.

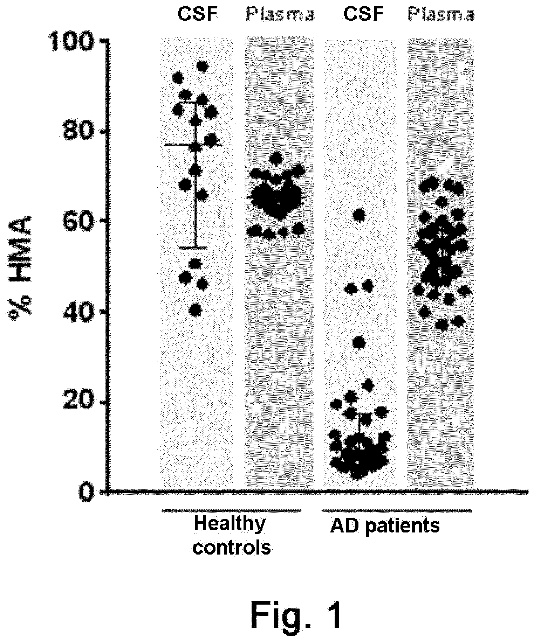

Fig. 1 shows the amount of HMA, by means of the peak height of HMA (as a percentage), obtained for samples of plasma and CSF from controls (healthy subjects, as defined above) and patients with AD. The ordinate axis ("y" axis) shows the peak height of HMA (as a percentage) and the abscissa axis ("x" axis) shows the group (the healthy controls on the left and the AD patients on the right). In addition, for each of the groups, the results obtained for the CSF samples are shown on the left and the results obtained for the plasma samples are shown on the right.

It is obvious to a person skilled in the art that if the content of HMA in CSF in AD patients is less than the content of HMA in CSF of healthy patients, the content of the two remaining fractions of albumin, according to the redox state of the Cys-34, i.e. of the HNA1 and/or the HNA2, will also be affected and can also serve as being indicative of AD.

### EXAMPLE

Example 1. Study of the diagnosis of Alzheimer's disease by measuring the content of HMA in CSF or the difference between said content and the content of HMA in plasma.

In the context of a multicentre, randomised, blind, controlled study in 42 patients who were being treated with therapeutic albumin and were diagnosed with mild-moderate AD, the present study was conducted to analyse the content of HMA in CSF and the difference between said content and the content of HMA in plasma.

For this, the content of HMA in baseline samples was measured (before the start of the clinical study) in the above-mentioned patients, both in CSF (N=34) and in plasma (N=37), as well as in the CSF (N=16) and plasma (N=37) of healthy subjects. The above-mentioned samples were in all cases obtained by lumbar puncture in the case of CSF and by drawing blood and then separating out the plasma in the case of the plasma samples, always following the standard medical procedures established for that purpose.

The samples of CSF and plasma mentioned were aliquoted and frozen at a temperature of -70ºC or below immediately after extraction. Before analysing the content of HMA, the samples were thawed at room temperature and, in the case of the plasma samples, the albumin concentration was determined by immunonephelometry or some other equivalent method. The HMA content was measured directly in the CSF samples whereas the plasma samples were diluted to a concentration of less than 10 mg/mL in phosphate buffer at pH 6.87. Next, the oxidised forms of the albumin were analysed by HPLC-FLD, as described above.

The results obtained are summarised in Fig. 1. As can be observed in said figure, the patients with AD show a great reduction in the content of HMA in CSF compared with the healthy subjects, whereas in plasma the reduction is slightly smaller. From the above, a deduction can be made of not only the diagnostic potential of the content of HMA in CSF but also the difference between said content and the content of HMA in plasma.

For AD patients, the median value obtained for the content of HMA in CSF is 9.6%, whereas the median of the content in plasma is 54.1%, the (HMA plasma/HMA CSF) ratio being 5.64. For healthy subjects, however, the median value of the content of HMA in CSF is 77.4% and that in plasma is 65.6%, the (HMA plasma/HMA CSF) ratio being 0.85.

## Claims

1. *In vitro* method for diagnosing Alzheimer's disease (AD), comprising the following steps:
a) determining the content of mercaptoalbumin (HMA) in a sample of cerebrospinal fluid (CSF); and
b) comparing the content determined in a) with the content of HMA in CSF in healthy subjects.

2. *In vitro* diagnostic method according to claim 1, **characterised in that** in step b), if the HMA content determined in step a) is less than that of the healthy subjects, it is indicative of AD.

3. *In vitro* diagnostic method according to claim 2, **characterised in that** in step b), if the HMA content determined in step a) is less than that of the healthy subjects, the patient is diagnosed with AD.

4. *In vitro* diagnostic method according to any one of the preceding claims, **characterised in that** the cut-off value of HMA content, below which it is considered to be indicative of AD and/or leads to a diagnosis of AD, is 37%.

5. *In vitro* diagnostic method according to any one of the preceding claims, **characterised in that** the sample comes from a human being.

6. *In vitro* diagnostic method according to claim 5, **characterised in that** said human being is an adult human being.

7. *In vitro* method for diagnosing Alzheimer's disease (AD), comprising the following steps:
a) determining the content of mercaptoalbumin (HMA) in a sample of cerebrospinal fluid (CSF) and in a sample of blood or plasma from a patient;
b) determining a difference or ratio between the content of HMA in the sample of CSF and the content of HMA in the sample of blood or plasma determined in step a); and
c) comparing the difference or ratio determined in step b) with the corresponding difference or ratio in healthy subjects.

8. *In vitro* diagnostic method according to claim 7, **characterised in that** in step b), the difference between the content of HMA in the sample of CSF and the content of HMA in the sample of blood or plasma determined in step a) is determined in the form of the ratio of the value of HMA in plasma / HMA in CSF (HMA plasma/HMA CSF).

9. *In vitro* diagnostic method according to claim 7, **characterised in that** in step c) of the method of the present invention, if the difference determined in step b) is greater than that of the healthy subjects, it is indicative of AD.

10. *In vitro* diagnostic method according to claim 9, **characterised in that** in step c) of the method of the present invention, if the difference determined in step b) is greater than that of the healthy subjects, the patient is diagnosed with AD.

11. *In vitro* diagnostic method according to any one of claims 7 to 10, **characterised in that** the cut-off value of the (HMA plasma/HMA CSF) ratio, above which it is considered to be indicative of AD and/or leads to a diagnosis of AD, is 1.1.

12. *In vitro* diagnostic method according to any one of claims 7 to 10, **characterised in that** the sample of blood or plasma is a sample of plasma.

13. *In vitro* diagnostic method according to any one of claims 7 to 10, **characterised in that** the sample comes from a human being.

14. *In vitro* diagnostic method according to claim 13, **characterised in that** said human being is an adult human being.

## Patentansprüche

1. *In-vitro*-Verfahren zum Diagnostizieren von Alzheimer-Krankheit (AD; für Englisch: "Alzheimer's disease"), umfassend die folgenden Schritte:
a) Bestimmen von dem Gehalt an Mercaptoalbumin (HMA) in einer Probe Liquor (CSF; für Englisch: "cerebrospinal fluid"); und
b) Vergleichen von dem Gehalt, der in a) bestimmt wird, mit dem Gehalt an HMA in CSF in gesunden Subjekten.

2. Diagnostisches *In-vitro*-Verfahren nach Anspruch 1, **dadurch gekennzeichnet, dass** in dem Schritt b), falls der HMA-Gehalt, der in dem Schritt a) bestimmt wird, geringer als der der gesunden Subjekte ist, dies für AD indikativ ist.

3. Diagnostisches *In-vitro*-Verfahren nach Anspruch 2, **dadurch gekennzeichnet, dass** in dem Schritt b), falls der HMA-Gehalt, der in dem Schritt a) bestimmt wird, geringer als der der gesunden Subjekt ist, der Patient mit AD diagnostiziert wird.

4. Diagnostisches *In-vitro*-Verfahren nach einem der vorhergehenden Ansprüche, **dadurch gekennzeichnet, dass** der Grenzwert des HMA-Gehalts, unter dem angenommen wird, dass es für AD indikativ ist und/oder zu einer Diagnose auf AD führt, 37 % beträgt.

5. Diagnostisches *In-vitro*-Verfahren nach einem der vorhergehenden Ansprüche, **dadurch gekennzeichnet, dass** die Probe aus einem Menschen stammt.

6. Diagnostisches *In-vitro*-Verfahren nach Anspruch 5, **dadurch gekennzeichnet, dass** der Mensch ein erwachsener Mensch ist.

7. Diagnostisches *In-vitro*-Verfahren zum Diagnostizieren von Alzheimer-Krankheit (AD), umfassend die folgenden Schritte:
a) Bestimmen von dem Gehalt an Mercaptoalbumin (HMA) in einer Probe Liquor (CSF) und in einer Probe Blut oder Plasma aus einem Patienten;
b) Bestimmen von einem Unterschied oder Verhältnis zwischen dem Gehalt an HMA in einer Probe CSF und dem Gehalt an HMA in der Probe Blut oder Plasma, die in a) bestimmt werden; und
c) Vergleichen von dem Unterschied oder Verhältnis, der oder das in dem Schritt b) bestimmt wird, mit dem entsprechenden Unterschied oder Verhältnis in gesunden Subjekten.

8. Diagnostisches *In-vitro*-Verfahren nach Anspruch 7, **dadurch gekennzeichnet, dass** in dem Schritt b) der Unterschied zwischen dem Gehalt an HMA in der Probe CSF und dem Gehalt an HMA in der Probe Blut oder Plasma, der in dem Schritt a) bestimmt wird, in der Form des Verhältnisses von dem Wert von HMA im Plasma/HMA im CSF (HMA Plasma/HMA CSF) bestimmt wird.

9. Diagnostisches *In-vitro*-Verfahren nach Anspruch 7, **dadurch gekennzeichnet, dass** in dem Schritt c) des Verfahrens der vorliegenden Erfindung, falls der Unterschied, der in dem Schritt b) bestimmt wird, größer als der der gesunden Subjekte ist, dies für AD indikativ ist.

10. Diagnostisches *In-vitro*-Verfahren nach Anspruch 9, **dadurch gekennzeichnet, dass** in dem Schritt c) des Verfahrens der vorliegenden Erfindung, falls der Unterschied, der in dem Schritt b) bestimmt wird, größer als der der gesunden Subjekte ist, der Patient mit AD diagnostiziert wird.

11. Diagnostisches *In-vitro*-Verfahren nach einem der Ansprüche 7 bis 10, **dadurch gekennzeichnet, dass** der Grenzwert für das (HMA Plasma/HMA CSF)-Verhältnis, über dem angenommen wird, dass es für AD indikativ ist und/oder zu einer Diagnose von AD führt, 1,1 beträgt.

12. Diagnostisches *In-vitro*-Verfahren nach einem der Ansprüche 7 bis 10, **dadurch gekennzeichnet, dass** die Probe Blut oder Plasma eine Probe Plasma ist.

13. Diagnostisches *In-vitro*-Verfahren nach einem der Ansprüche 7 bis 10, **dadurch gekennzeichnet, dass** die Probe aus einem Menschen stammt.

14. Diagnostisches *In-vitro*-Verfahren nach Anspruch 13, **dadurch gekennzeichnet, dass** der Mensch ein erwachsener Mensch ist.

## Revendications

1. Méthode *in vitro* pour le diagnostic de la maladie d'Alzheimer (AD), comprenant les étapes suivantes :
a) déterminer la teneur en mercaptalbumine (HMA) dans un échantillon de fluide cérébrospinal (CSF) et
b) comparer la teneur déterminée en a) avec la teneur en HMA dans du CSF de sujets sains.

2. Méthode de diagnostic *in vitro* selon la revendication 1, **caractérisée en ce que** lors de l'étape b), si la teneur en HMA déterminée lors de l'étape a) est inférieure à celle des sujets sains, c'est un indicateur de AD.

3. Méthode de diagnostic *in vitro* selon la revendication 2, **caractérisée en ce que** lors de l'étape b), si la teneur en HMA déterminée lors de l'étape a) est inférieure à celle des sujets sains, le patient est diagnostiqué atteint de AD.

4. Méthode de diagnostic *in vitro* selon l'une quelconque des revendications précédentes, **caractérisée en ce que** la valeur seuil de teneur en HMA, en dessous de laquelle elle est considérée indicative de AD et/ou conduit à un diagnostic de AD, est 37%.

5. Méthode de diagnostic *in vitro* selon l'une quelconque des revendications précédentes, **caractérisée en ce que** l'échantillon provient d'un être humain.

6. Méthode de diagnostic *in vitro* selon la revendication 5, **caractérisée en ce que** ledit être humain est un être humain adulte.

7. Méthode de diagnostic in vitro pour diagnostiquer la maladie d'Alzheimer (AD), comprenant les étapes suivantes :
a) déterminer la teneur en mercaptalbumine (HMA) dans un échantillon de fluide cérébrospinal (CSF) et dans un échantillon de sang ou de plasma provenant d'un patient ;
b) déterminer une différence ou un ratio entre la teneur en HMA dans l'échantillon de CSF et la teneur en HMA dans l'échantillon de sang ou de plasma déterminées lors de l'étape a) ; et
c) comparer la différence ou le ratio déterminé lors de l'étape b) avec la différence ou le ratio correspondant chez des sujets sains.

8. Méthode de diagnostic in vitro selon la revendication 7, **caractérisée en ce que** lors de l'étape b), la différence entre la teneur en HMA dans l'échantillon de CSF et la teneur en HMA dans l'échantillon de sang ou de plasma déterminées lors de l'étape a) est déterminée sous la forme du ratio de la valeur de HMA dans le plasma / HMA dans le CSF (HMA plasma/HMA CSF).

9. Méthode de diagnostic in vitro selon la revendication 7, **caractérisée en ce que** lors de l'étape c) de la méthode selon la présente invention, si la différence déterminée lors de l'étape b) est supérieure à celle des sujets sains, c'est un indicateur de AD.

10. Méthode de diagnostic in vitro selon la revendication 9, **caractérisée en ce que** lors de l'étape c) de la méthode selon la présente invention, si la différence déterminée lors de l'étape b) est supérieure à celle des sujets sains, le patient est diagnostiqué atteint de AD.

11. Méthode de diagnostic in vitro selon l'une quelconque des revendications 7 à 10, **caractérisée en ce que** la valeur seuil du ratio (HMA plasma/HMA CSF), au-dessus de laquelle il est considéré comme indicatif de AD et/ou conduit à un diagnostic de AD, est 1.1.

12. Méthode de diagnostic in vitro selon l'une quelconque des revendications 7 à 10, **caractérisée en ce que** l'échantillon de sang ou plasma est un échantillon de plasma.

13. Méthode de diagnostic in vitro selon l'une quelconque des revendications 7 à 10, **caractérisée en ce que** l'échantillon provient d'un être humain.

14. Méthode de diagnostic in vitro selon la revendication 13, **caractérisée en ce que** ledit être humain est un être humain adulte.
